# EUROPEAN PATENT SPECIFICATION

(11) **EP 4 045 111 B1**
(45) Date of publication and mention of the grant of the patent: **25.11.2026**
(21) Application number: 20877277.2
(22) Date of filing: 15.10.2020
(51) Int. Cl.: A61M 5/14, A61M 5/142, A61M 5/145, A61M 5/148, A61M 5/168, A61M 5/172, A61B 5/00, A61B 5/145, F04B 43/04, F04B 45/047, F04B 49/06

(54) **DEVICE FOR DELIVERING MEDICATION TO A PATIENT**
VORRICHTUNG ZUM ABGEBEN VON MEDIKAMENTEN AN EINEN PATIENTEN
DISPOSITIF POUR ADMINISTRER UN MÉDICAMENT À UN PATIENT

(30) Priority: 18.10.2019 US 201962923099 P
(43) Date of publication of application: 24.08.2022
(73) Proprietor: Aita Bio Inc., New York, NY 10010 (US)
(72) Inventor: SMEYS, Peter, San Jose, CA 95120 (US); JOHNSTON, David, Libertyville, IL 60048 (US); MENTURE, Jacob, New York, NY 10018 (US)
(74) Representative: Modiano, Gabriella Diana
(86) International application number: PCT/US2020/055845
(87) International publication number: WO 2021/076806

(56) References cited:
- US-A- 6 106 245
- US-A1- 2004 019 331
- US-A1- 2004 019 331
- US-A1- 2006 283 465
- US-A1- 2008 234 630
- US-A1- 2011 054 285
- US-A1- 2015 025 503
- US-A1- 2015 258 273
- US-A1- 2019 125 967
- US-A1- 2019 151 639

## Description

### REFERENCE TO RELATED APPLICATIONS

This application claims priority to U.S. provisional application number 62/923,099, filed on October 18, 2019 entitled "Device For Delivering Medication To a Patient".

### FIELD OF THE INVENTION

The present invention relates to a device for delivering medication to a patient.

### BACKGROUND OF THE INVENTION

Various infusion systems exist that utilize devices for delivering liquid medication or other therapeutic fluid to patients subcutaneously. For patients with diabetes mellitus, for example, conventional infusion systems incorporate various pumps that are used to deliver insulin to a patient. These pumps have the capability of delivering assorted fluid delivery profiles which include specified basal rates and bolus requirements. For example, these pumps include a reservoir to contain the liquid medication along with electromechanical pumping technology to deliver the liquid medication via tubing to a needle that is inserted subcutaneously into the patient.

Although such conventional pumps/infusion systems are adequate for their intended purpose, such pumps have difficult controlling drug delivery precisely thereby causing harm to the patient. That is, these pumps have large stroke volumes resulting in inaccurate basal rate infusion and incorrect insulin dosing. Further, with these infusion systems, diabetes patients must install and carry at least two bulky and obtrusive devices on their bodies. This causes significant inconvenience for the patient during his/her daily activities.

Exemplary drug delivery devices are known from US 2004/019331, US 2019/125967, US 2006/283465, and US 2011/054285, however, it would be advantageous to provide further improved infusion system over these conventional infusion systems.

### SUMMARY OF THE INVENTION

The claimed invention lies in a device for delivering a medication to a patient according to appended independent claim 1. Further embodiments are given in the appended dependent claims.

### BRIEF DESCRIPTION OF DRAWINGS

Fig. 1 depicts a block diagram of an example drug infusion system for infusing medication to a patient.
Fig. 2A depicts a detailed schematic block diagram of the infusion system in Fig. 1.
Fig. 2B depicts another schematic block diagram of infusion system 200 in Fig. 1.
Figs. 3A-3C depict cross-sectional views of example pumping sequences of MEMS devices in series that function as the pumping unit in Fig. 1.
Figs. 3D-3F depict cross-sectional views of an example MEMS device (in various configurations) as part of the pumping unit in Fig. 1.
Figs. 3G-3I depict cross-sectional views of another example MEMS device as the pumping unit in various configurations, not covered by the claimed invention.
Figs. 3J, 3L and 3N depict cross-sectional views of other example MEMS devices as the pumping units in various configurations, not covered by the claimed invention.
Fig. 3K depicts a top view of a valve in Fig. 3J along the line 3K-3K.
Fig. 3M depicts a top view of a valve in Fig. 3L along the line 3M-3M.
Figs. 4A and 4B depict cross sectional views of an example MEMS device functioning as a valve (in various configurations) in the pumping unit in Fig. 1.
Figs. 5A and 5B depict block diagrams of example configurations of the pumping unit in Fig. 1 to vary flow rate and pressure, respectively.
Fig. 6 depicts a cross sectional view of an example device (or pod) for delivering insulin to a diabetes patient.
Fig. 7 depicts a cross sectional view of another example device (or pod) for delivering insulin to a diabetes patient.

### DETAILED DESCRIPTION OF THE INVENTION

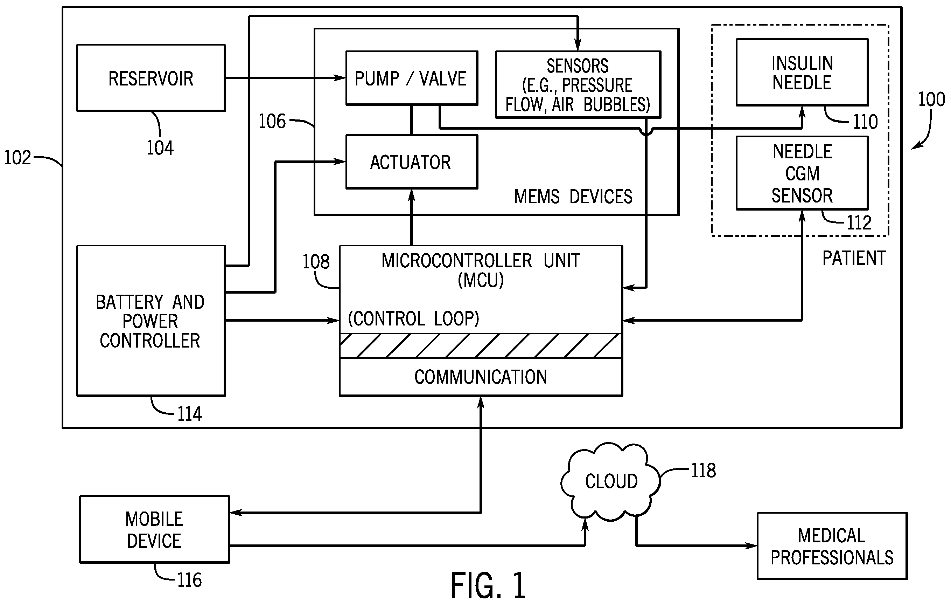

Fig. 1 depicts a block diagram of an example drug infusion system 100 for infusing a drug (i.e., medication) or other fluid to a patient, i.e., a user of drug infusion system such as system 100). In this example, infusion system 100 is configured to infuse insulin to a patient for diabetes management (e.g., type 1). However, system 100 can be configured to infuse other medications such as small molecule pharmaceutical solutions, large molecule or protein drug solutions, saline solutions, blood or other fluids known to those skilled in the art.

Infusion system 100 includes device 102 (or pod) for delivering insulin to a diabetes of other fluid medication to a patient. In this configuration, device 102 incorporates micro-electro-mechanical systems (MEMS) devices into its architecture for motive force and sensing functionality (as described in more detail below). Among other benefits, the MEMS technology (layers) in device 102 architecture enables direct connection between fluid path components for infusion without any tubing, connectors and/or separate mechanical valves. As a result, device 102 not only produces greater precision in pumping volume, it requires less power for operation, has better reliability, and less drug waste held up in fluid pathways (smaller dead volume). (In addition, MEMS manufacturing results in a very accurate device dimension with low tolerance (e.g, 1um)). Consequently, device 12 may be fabricated to a significantly smaller scale. In short, by incorporating MEMS technology, device 102 is configured as a fully autonomous and integrated wearable unit or apparatus for diabetes management in which continuous glucose monitoring (CGM), insulin delivery and closed loop control are provided together to ensure insulin is delivered at very precise rates. Device 102 details appear below. (Note that MEMS devices are also known as microsystem technology and micromachined devices).

Device 102 includes reservoir 104, pumping unit or element 106 (also may be referred to as a micropump), microcontroller unit (MCU) 108, insulin delivery needle 110, glucose monitoring components 112 including the CGM (device), a sensor and needle (percutaneously inserted in the patient), and battery and power controller 114. CGM, as known to those skilled in the art, tracks patient glucose levels and permits those levels to be used in algorithms that control flow rate. MCU 108 controls the operation of pumping unit 106 as described below.

Reservoir 104 is configured to receive and store insulin for its delivery over a course of about three days, or as needed. However, reservoir size may be configured for storing any quantity of fluid as required.

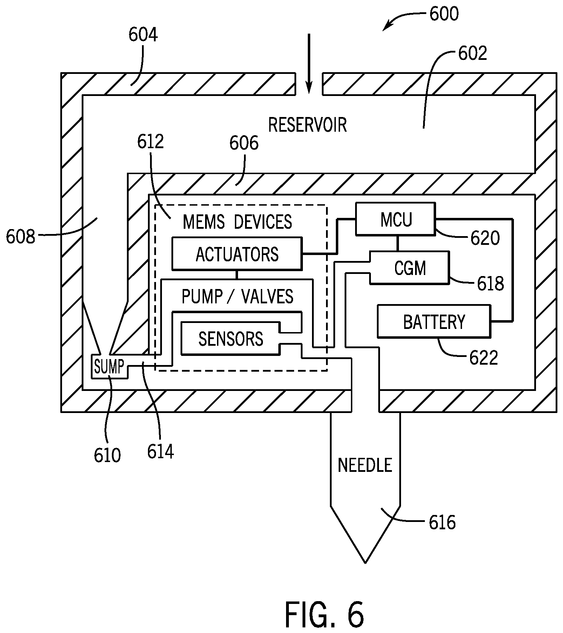

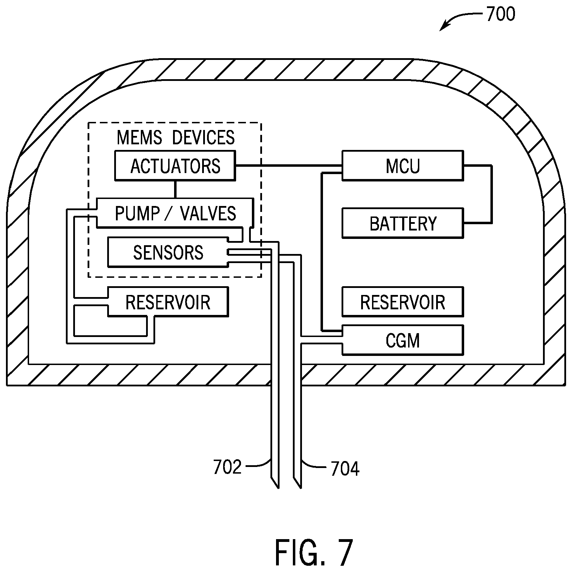

Pumping unit 106 fluidly communicates with reservoir 104 to enable infusion as needed. In one example configuration, pumping unit 106 may connect directly to reservoir 104 by bonding and/or adhesive with corresponding holes in alignment. In another example configuration, a short interposer may be used as a connector. In practice, the interposer functions as a funnel. The interposer consists of two plates of glass and/or silicon bonded together to form a plurality of holes and channels which may be employed to transition one size opening to another between pumping unit 106 to reservoir 104. These are examples. Those skilled in the art know that other connector options may be used to achieve desired results. Pumping unit 106 also fluidly communicates with insulin needle 110 for insulin delivery. Insulin needle 110 and/or a cannula itself surrounding needle 110 may be inserted directly into pumping unit 106. Alternatively, a similar interposer may also be used to connect needle 110 or cannula to pumping unit 106. In this configuration, a plate covering the larger end of the interposer includes several holes to ensure proper fluid transfer in any orientation. Examples of these connections are shown and described below with respect to Figs. 6 and 7.

Pumping unit 106 incorporates MEMS devices that function as a pump for pumping fluid such as insulin, valves for regulating flow, actuators for moving or controlling the pump and valves, and sensors for sensing pressure, insulin flow, presence of air in the fluid path and across the channels in the MEMS devices. In one example configuration, the MEMS devices are each a piezoelectric transducer (or other MEMS devices including capacitive transducers or piezoresistive transducers) that acts as the active element for pumping fluid, but other MEMS structures or technology may be used to achieve desired results as known to those skilled in the art. Operation and functional details of the MEMS devices (e.g., piezoelectric transducer) appear in more detail below.

MCU 108 electronically communicates with the actuator and sensors in pumping unit 106 as well as the CGM sensor, as the monitoring components 112 in Fig. 1. Among several functions, MCU 108 operates to control the operation of pumping unit 106 to deliver insulin through insulin needle 110 from reservoir 104 at specific doses, i.e., flow rates over specified time intervals, based on CGM data converted to desired flow rate via control algorithms. As part of this, MCU 108 also functions to perform dual closed control loop (also referred to as loop control) in which a flow rate is (1) prescribed by the control algorithm via data from the CGM and (2) subsequently monitored to precisely maintain such flow rate. (The sensors on pumping unit 106 measure pressure, flow rate and presence of air or other gas bubbles to ensure that flow rate is accurately maintained.) Control algorithms for such control reside in memory of MCU 108. Dual closed loop control is described in more detail below. Importantly, once device 102 is started, it operates as a fully autonomous and integrated unit for diabetes management without connection or tether to a mobile device or computer. MCU 108 will incorporate sufficient memory known to those skilled in the art to store all data collected and generated (e.g., glucose levels, flow rates, dose delivered, pressures, faults etc.) for up to 3 days or more as desired. Once in proximity to (or commanded by) a mobile device or computer, the data stored may be uploaded to such mobile device or computer (e.g., via Bluetooth, WIFI, wired connection). This is described in more detail below.

Battery and power controller 114 controls the power to MCU 108 and pumping unit 106 to enable those components to function properly as known to those skilled in the art. The CGM is powered by battery and power controller 114 through MCU 108.

Infusion system 100 further includes mobile device 116 that wirelessly communicates with the communications circuitry on an ASICs chip along with MCU 108. Communication circuitry communicates with the MCU 108 as known to those skilled in the art. Communication may be achieved using Bluetooth, WIFI, NFC or other means of communication known to those skilled in the art. The application on mobile device 116 wirelessly communicates with one or more medical professionals via cloud 118 (via cellular, WIFI or other) as known to those skilled in the art. An application on mobile device 118 functions to receive, analyze and visualize data generated by device 102. The application will upload any configurations, settings and firmware updates when paired with device 102 upon startup. The application on mobile device 116 will also send data to cloud 118.

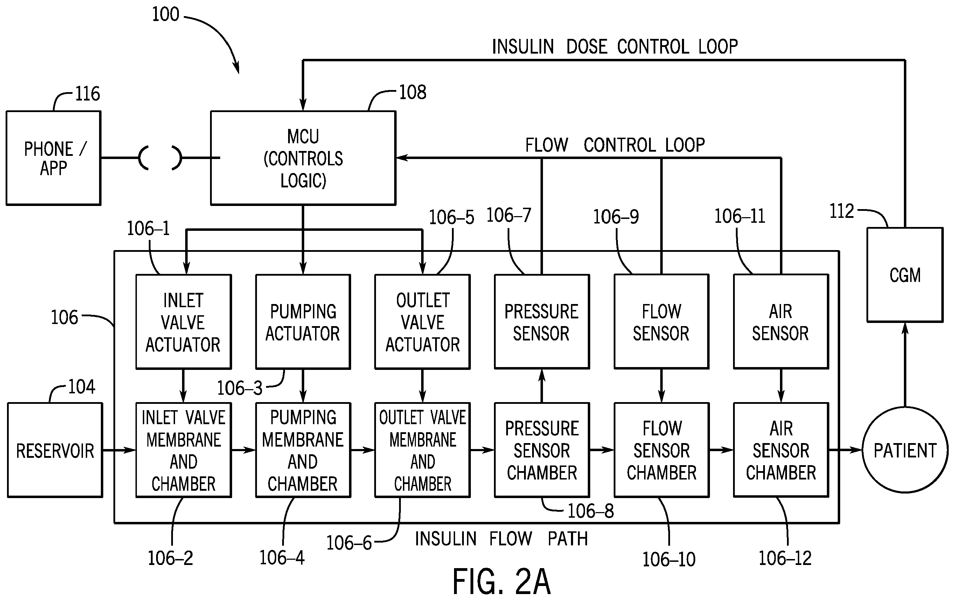

Fig. 2A depicts a detailed schematic block diagram of infusion system 100 in Fig. 1 in which two control loops (within a medication delivery device) are illustrated in connection with MCU 108, CGM and pumping unit 106 of the delivery device - (1) an insulin dosing control loop and (2) flow control loop as described below in detail.

In Fig. 2A, pumping unit 106 of the delivery device includes reservoir 104, pumping unit 106, MCU 108 and CGM (part of glucose monitoring component 112) as shown. Pumping unit 106 incorporates an inlet valve including inlet valve actuator 106-1 and inlet valve membrane and chamber 106-2, a pump or pumping section including pumping actuator 106-3 and pumping membrane and chamber 106-4 and an outlet valve including outlet valve actuator 106-5 and outlet valve membrane and chamber 106-6. Pumping unit 106 further incorporates sensors including pressure sensor 106-7, pressure sensor chamber 106-8, flow sensor 106-9 and flow sensor chamber 106-10, air sensor 106-11 and air sensor chamber 106-12. The pumping unit 106 is used for pumping fluid such as insulin, valves for regulating flow, actuators for moving or controlling the pump and valves, and sensors for sensing pressure, insulin flow, presence of air in the fluid path and across the channels in the MEMS devices. Further details are described below.

In the first instance, the insulin dosing control loop automatically monitors and determines (i.e., sets) a proper flow rate for the patient. Specifically, CGM (monitoring components 112) tracks (measures) patient glucose levels which are converted to instructions for flow rate by control algorithms as known to those skilled in the art. The control algorithms residing on MCU 108 controls and commands the pumping unit 106 (MEMS devices as valves and pumping elements as described below) to deliver insulin through insulin needle 110 from reservoir 104 at that flow rate, based on the CGM converted data. This represents the first control loop which utilizes MCU 108, CGM and pumping unit 106.

In the second instance, the control loop actively and automatically monitors the actual flow rate to ensure that the set flow rate is delivered precisely as originally commanded, i.e., to ensure that the actual flow rate monitored is the same as the flow rate commanded. As the first step, MCU 108 will set an initial flow rate as determined above, i.e., a voltage and frequency, as well as a driving waveform, for a patient and the pumping unit 106 will pump at that flow rate. Next, a flow sensor measures the actual flow rate (Q) off pumping unit 106 (MEMS device as described herein). At the same time, CGM (monitoring component 112) measures the glucose level in the patient and converts that glucose level to a flow rate (S). Then, MCU 108 will then compare the flow rate (S) with the actual flow rate (Q) sensed. That is, MCU 108 will measure (calculate) the difference ((S - Q)) (also known as an error signal) and command pumping unit 106 to adjust the initial flow rate by changing the voltage and/or frequency of pumping unit 106. This operation will automatically continue, in a looping fashion, until the error signal is reduced to zero. Once at zero, pumping unit 106 will continue pumping at the same flow rate until alternate rate instructions come from the control algorithms residing on the MCU. In short, pumping unit 106 will increase or decrease voltage and/or frequency to adjust flow rate from pumping unit 106 (MEMS device) in accordance with the error signal generated. This represents the second control loop which utilizes MCU 108, CGM, pumping unit 106 (and flow sensor as part of pumping unit 106).

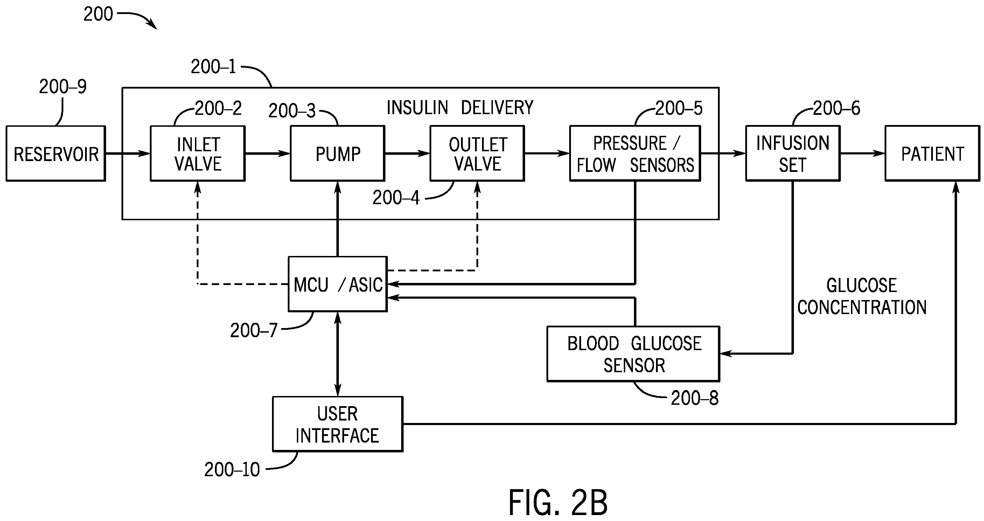

Fig. 2B depicts another schematic block diagram of infusion system 200 in Fig. 1 in which internal components of a device for delivering insulin or other fluid medication are shown. As described above, the delivery device includes pumping unit 200-1 incorporating inlet valve 200-2, pump 200-3, outlet valve 200-4 and pressure/flow sensor(s) 200-5. Similar to other examples described herein, pumping unit 200-1 incorporates MEMS devices that functions as these components. The delivery device further includes reservoir 200-9, infusion set 200-6 and blood glucose sensor 200-8 (or glucose monitoring device as described above), microcontroller unit (MCU) 200-7 and user interface 200-10.

Insulin is initially stored in reservoir 200-9 and delivered through inlet valve 200-2, pump 200-3 (pumping chamber), outlet valve 200-4, pressure sensors 200-5 with at least one hydraulic resistor and infusion set 200-6. The valves may be active valves controlled by MCU 200-7 (or ASIC), or the valves may be passive valves such as check valves without a driver known to those skilled in the art. Pump 200-3 is driven by electronics to withdraw insulin from reservoir 200-9 through inlet valve 200-2, and to pump insulin out of the pump 200-3 to infusion set 200-6 through outlet valve 200-4. The delivery flow rate is captured by pressure sensors 200-5, as well as the occlusion detection is sensed by at least one pressure sensor. This is similarly described above with respect to an embodiment above.

Also similar to the embodiment in Fig. 2A, this delivery device includes dual control loops for precise dosing control. Specifically, the actual flow rate is controlled by a first feedback control loop, wherein the measured flow rate is used to compensate for the pump actuation voltage or frequency to maintain the same stroke volume/ flow rate. The set flow rate is then controlled by a second feedback control loop wherein the flow rate is determined using the glucose level measured by blood glucose sensor to maintain a precise glucose level. Patients can also manually control the flow rate on manual mode using the user interface.

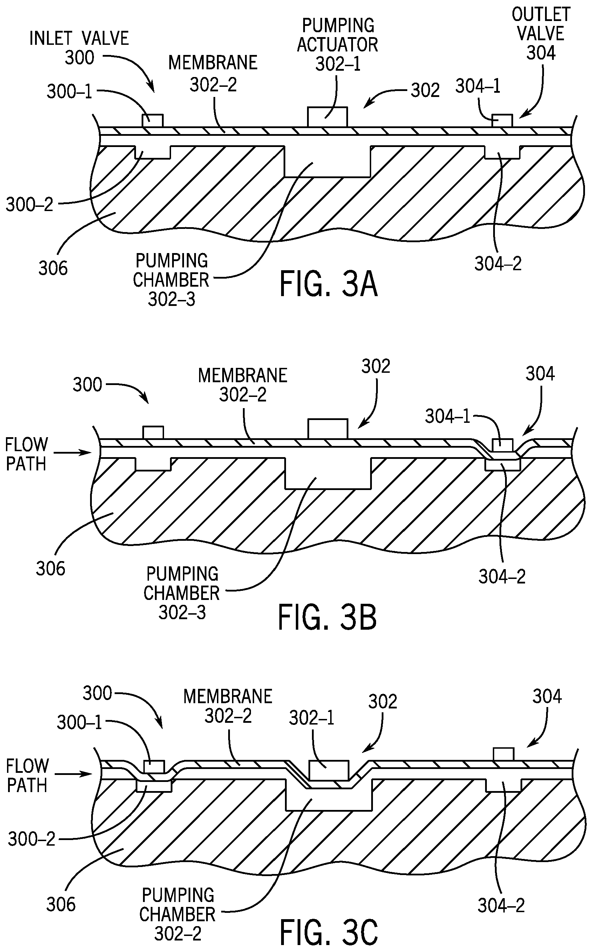

Figs. 3A-3C depict example pumping sequences of MEMS devices in series (as pumping unit 106). The MEMS devices are in series include inlet valve 300, pumping section 302 and outlet valve 304. Inlet valve 300 includes inlet valve actuator 300-1 and inlet valve chamber 300-2 and membrane 302-2. Pumping section 302 includes pumping actuator 302-1, pumping membrane 302-2 and chamber 302-3. Chamber 302-3 is defined in part by a bottom substrate 306 and pumping membrane 302-2 which sits between chamber 302-3 and pumping actuator 302-1. Outlet valve 304 includes outlet valve actuator 302-1, outlet valve chamber 302-2 and membrane 302-2. In Fig. 3A, inlet valve 300, pumping section 304 (actuator and pumping chamber) and outlet valve 302 are shown in neutral, starting position with no power. In this configuration, membrane 302-2 is in a neutral position.

Inlet valve 300, pumping section 302 and outlet valve 304 function together to withdraw insulin (or other fluid) from reservoir 104 and drive the insulin at selected rates to the patient dictated by the CGM. Operation is described as follows. As shown in Fig. 3B, outlet valve 302 is in an energized configuration where pumping membrane 302-2 is depressed (i.e., forced downwardly). Then, pumping actuator 302-1 causes the pumping membrane 302-2 and chamber 302-3 to move the insulin by lowering the chamber pressure as membrane 302-2 is withdrawn from the chamber 302-3 (by the pumping actuator under negative voltage).

Next, inlet valve 300 is closed by the inlet valve actuator 302-1, by changing the position of the inlet valve membrane onto an opposing surface or edge. Then, pumping membrane 302-2 is moved back into pumping chamber 302-2 by pumping actuator 302-1 driven in that direction, thereby increasing pressure in pumping chamber 302-3. With pumping membrane 302-2 sweeping the volume of pumping chamber 302-3, thereby increasing pressure therein, outlet valve 304 is opened with outlet valve actuator 302-1. This is shown in Fig. 3C. This permits fluid to flow out and into a channel carrying the fluid toward the patient.

At the exit region of the MEMS devices, pressure sensor and flow sensor sense pressure and flow as identified in Fig. 2. In one example, the MEMS devices (piezoelectric elements) will sense pressure and flow by sensing both pressure and pressure drop due to flow across the membranes that transmit pressure as a force to the piezoelectric elements as known to those skilled in the art. However, other methods may be used to sense pressure and/or flow as known to those skilled in the art.

In addition to pressure and flow sensing, air bubble sensing is performed in the flow path to determine if bubbles are present in the fluid path. In an example configuration, the air sensor uses an ultrasonic device to measure time-of-flight for sound waves changing velocity of transmission between air and insulin (fluid) solution. That is, once air volume is determined, it is subtracted from the volume of insulin dosage to alert and/or adjust for lack of dosing.

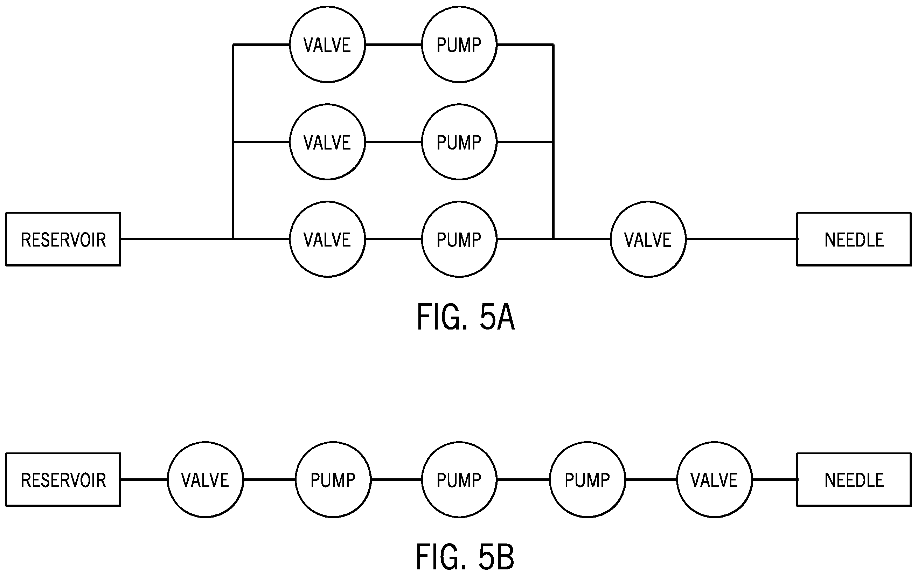

These sensors will transmit information to MCU 108 and processed via software to provide real-time adjustments to fluid flow and pressure as needed per patient as well as alert/alarm signals for conditions present. The adjustments in flow rate may be accomplished in two ways. First, flow rate may be adjusted by increasing pulse frequency of the actuators in the inlet, pumping and outlet chambers. Second, flow rate may be adjusted by increasing the voltage of each pulse to the pumping actuator. This increase in voltage ultimately causes an increase in amplitude of deflection of the pumping membrane. These real-time adjustments in flow will yield significantly improved flow precision, pulsatility and accuracy. However, there are other ways to adjust flow and/or pressure. Examples of this appear in Figs. 5A and 5B. In brief, the pumping chambers (of MEMS devices) may be configured in parallel to increase flow rate and/or pumping chambers may be configured in series to increase output pressures as required per patient. Details are described below.

As for alerts and alarms, MCU 108 may also provide signals for alarm/alert conditions such as low flow and excess pressure (both indicating occlusion for example) causing a potential under-dosing of the patient. In addition, the alarm/alert will alert of conditions such as presence of air indicating absence of drug dosing or possibly an empty reservoir/end of dose, as well as conditions of flow in excess of that set by the insulin dosing control algorithm.

As described above, inlet and outlet valve actuators 300, 304 are shown and described as an active design in which the inlet and outlet valve actuators are configured with moving parts, i.e., a piezoelectric transducer actuator retracts a membrane to enable fluid flow. In the passive design, the inlet valve actuator and outlet valve actuator function passively. That is, the inlet valve actuator and outlet valve actuator are configured without moving parts such as in a diffuser valve. In essence, the inlet and outlet valve actuators are considered not be present. Passive valves are described below in more detail.

Also note, that inlet valve 300 and outlet valve 304 are MEMS devices shown are sized and configured (i.e., fabricated) similarly. MEMS device 302, as the pumping section, is configured with a larger chamber than the chambers of the inlet and outlet valves. This is one example configuration for these MEMS devices. However, MEMS devices 300, 302, 304 may be configured and fabricated to various sizes and/or constructions to achieve desired pressure and flow rate as known to those skilled in the art.

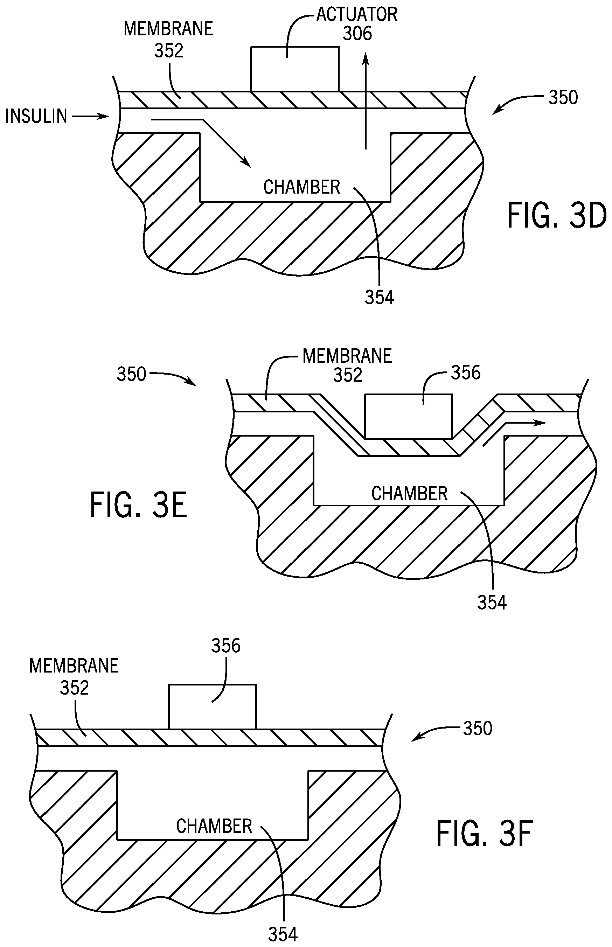

Figs. 3D-3F depict cross sectional views of an example MEMS device 350 as the pumping unit in various configurations. In Fig. 3D, membrane 352 returns to a neutral position from a previous compressed position when voltage applied goes to zero. The membrane 352 may be withdrawn to a position above neutral when negative voltage is applied to actuator 356. Both actions thereby reduce chamber 304 pressure and drawing insulin from inlet. Applying the negative voltage may reduce the chamber pressure more than the zero voltage/neutral position, and increase the volume drawn into the pumping chamber 354. In Fig. 3E, actuator 356 is energized with positive voltage (e.g., 20V-200V), membrane 352 is driven into chamber 354, thereby increasing pressure and flow out of chamber 354. In Fig. 3F, membrane 352 is in a neutral position and the actuator is in a non-energized position.

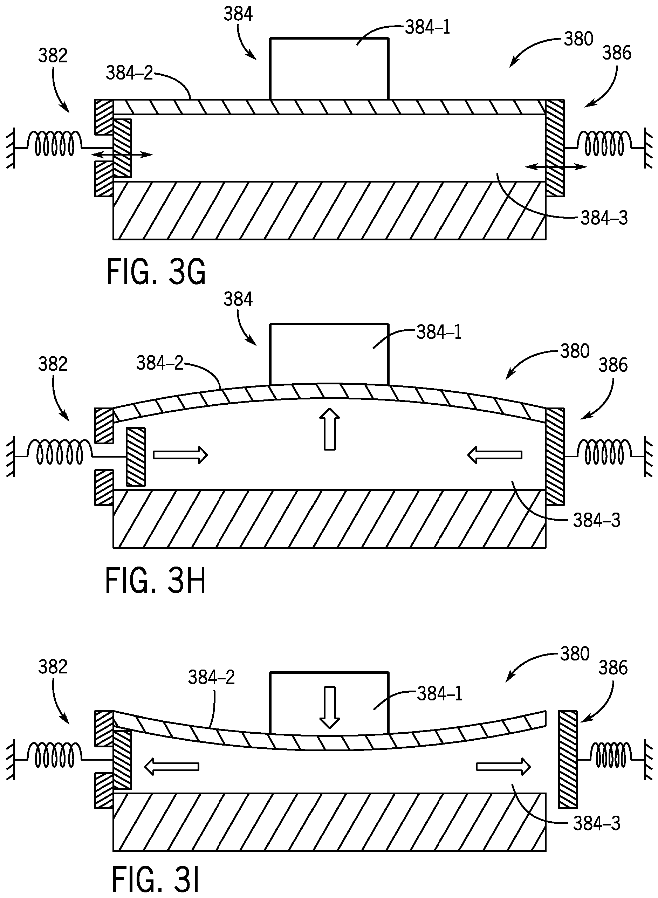

Figs. 3G-3I depict cross sectional views of example MEMS device 380 as the pumping unit in various configurations, not covered by the claimed invention, with inlet valve 382, pumping section 384 and outlet valve 386 in series. These valves shown in Figs. 3G-3I are representations of those valves which depict spring loaded drives as part of those valves.

In Fig. 3G for example, membrane 384-2 is in a neutral position and the actuator is in a non-energized position. Pumping section 384 includes pumping actuator 384-1, membrane 384-2 and chamber 384-3 as shown. Inlet and outlet valves 382 and 386 each include mechanical ("passive") check valves.

In Fig. 3H for example, MEMS device 380 is in an activated position from the previous neutral position. In brief, during suction operation, pumping actuator 384-1 is driven to enable higher chamber volume which creates a negative pressure in the chamber to open the inlet valve 382. In Fig. 3H, actuator 384 causes membrane 384-2 to bend upwardly (bowing). This creates a negative pressure in chamber 384-3. As a result, suction is created whereby the pressure increases valve 386 closure while the pressure opens valve 382 even greater. Specifically, membrane 384-2 may be withdrawn to a position above neutral when negative voltage is applied to actuator 384-1. Both actions thereby reduce chamber 384-3 pressure and drawing insulin within the chamber by way of a one way inlet flow valve 382. Applying the negative voltage may reduce the chamber pressure more than the zero voltage/neutral position, and increase the volume drawn into the pumping chamber 384-3.

In Fig. 3I for example, pumping actuator 384-1 is energized with positive voltage (e.g., 20V-200V), membrane 384-2 is driven into chamber 384-3, thereby increasing pressure and flow out of chamber 384-3 via outlet valve 384. That is, during the pumping operation, pumping section 384 is driven to have a smaller chamber volume which creates a positive pressure in the chamber to open outlet valve 386.

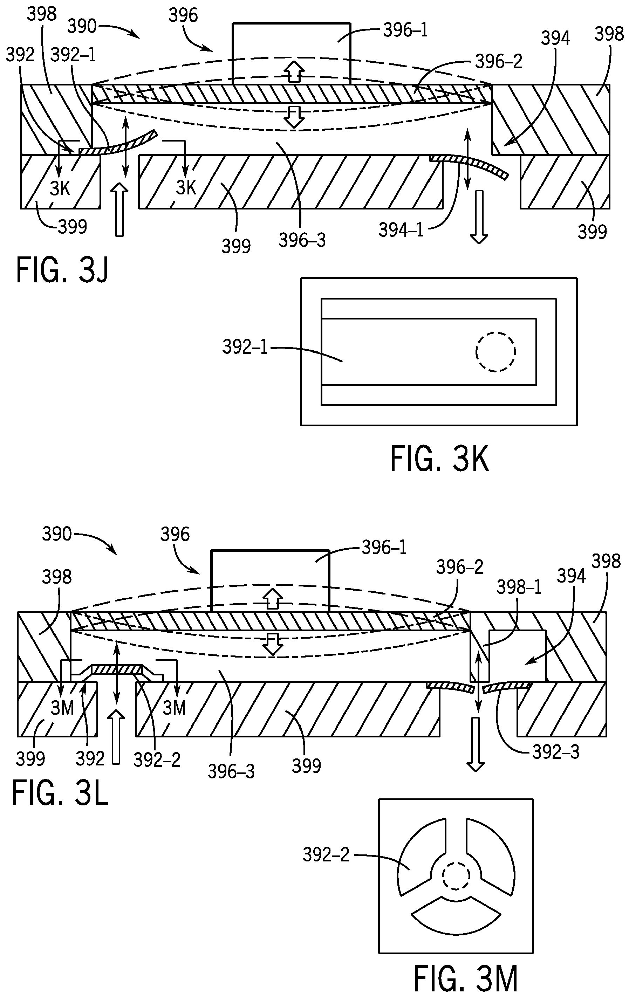

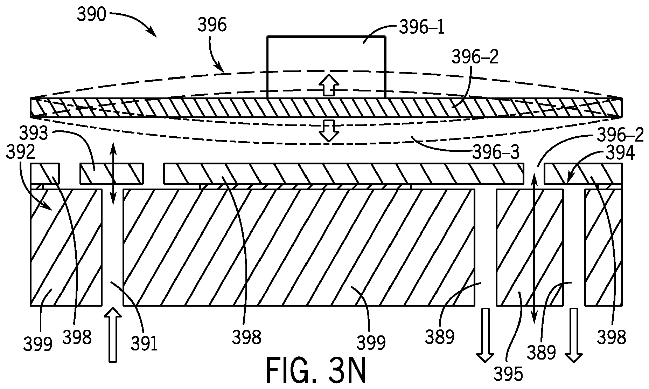

Figs. 3J, 3L and 3N depict cross-sectional views of other example MEMS devices as pumping units in various configurations, not covered by the claimed invention. These examples are similar to those in Figs. 3G-3I functionally, except the valving mechanisms are structurally different.

In Fig. 3J specifically, pumping unit 390 as shown includes pumping section 396 and two valves 392, 394 that are used for controlling flow into and out of pump chamber 396-3 (of pump 396), as pump actuator 396-1 moves membrane 396-2. Membrane 396-2 is positioned between parts of substrate 398 of pumping unit 390 to enable it to bow. Membrane 396-2 and substrate 398 may be an integral piece or secured by bonding or other means known to those skilled in the art. Inlet and outlet values 392, 394 include flaps 392-1 and 394-1, respectively. Theses flaps are secured to wall of the pumping unit 390 as an integral piece or as a bonded or attached piece as known to those skilled in the art. Specifically, flap 392-1 is secured or positioned in an opening or channel defined by an edge in wall of substrate 398 (of pumping unit 390) and substrate 399 as shown. Flap 394-1 is secured to the edge of substrate 399. These flaps will bend or bow into and out of chamber 396-3 as shown. Fig. 3K depicts a top view of valve 392 (along line 3K-3K in Fig. 3J illustrating flap 392-1) in more detail. The MEMs device described is made of semiconductor materials as known to those skilled in the art (e.g., Silicon and Silicon Dioxide).

In brief, during suction operation, pumping section 396 is driven to enable higher chamber volume which creates a negative pressure in the chamber to open the inlet flap valve 392-1. During the pumping operation, pumping section 396 is driven to have a smaller chamber volume which creates a positive pressure in the chamber to open outlet flap valve 394. Fluid will flow through inlet valve 392 and out outlet valve 394 (by way of flaps 392-1 and 394-1) as shown by the arrows.

In Fig. 3L, pumping unit 390 as shown includes pumping section 396 and two valves 392, 394, as described above, that are used for controlling fluid medication flow into and out of pump chamber 396-3 (of pump 396), as pump actuator 396-1 moves membrane 396-2. In this example, inlet value 392 includes an expandable (stretchable) element 392-2 that expands or moves (stretches) off of an opening into chamber 396-3 under pressure from force within chamber 396-3 as membrane 396-2 moves. Outlet valve 394 includes dual flaps 392-3 that bend or bow into an exit channel to enable fluid medication to exit chamber 396-3. Flaps 392-3 rest against post 398-1 of substrate 398 in neutral or resting position.

The expandable element and dual flaps of these valves 392, 394 are secured to wall of the pumping unit 390 as an integral piece or as a bonded or attached piece as known to those skilled in the art. The expandable element and flaps will bend or bow off of an opening to enable liquid (fluid) medication to move into and out of chamber 396-3 (through channels as shown by arrows) of pump 396 as shown. Fig. 3M depicts a top view of the valve 392 with expandable element 392-2 in Fig. 3L, along the line 3M-3M, in more detail.

In Fig. 3N, pumping unit 390 as shown similarly includes pumping section 396 and inlet and outlet valves 392, 394 that are used for controlling flow into and out of pumping chamber 396-3 (via channels 391, 389 in substrate 399 of pumping unit), as pumping actuator 396-1 moves membrane 396-2 with respect to substrate 398 of pumping unit 390. Inlet and outlet values 392, 394 include pistons 393, 395, respectively, that cover openings in substrate 398 that lead to channels 391, 389 as shown. Similar to the passive valves described with respect to the other embodiments above, posts or pistons 393, 395 move accordingly to enable flow as pressure is increased and decreased in pumping chamber 396-3. The posts/pistons may be secured, for example, using preloaded components to drive the piston/posts upwardly or downwardly as needed or any other mechanism known to those skilled in the art. The MEMs device described are made of semiconductor materials as known to those skilled in the art (e.g., Silicon and Silicon Dioxide.

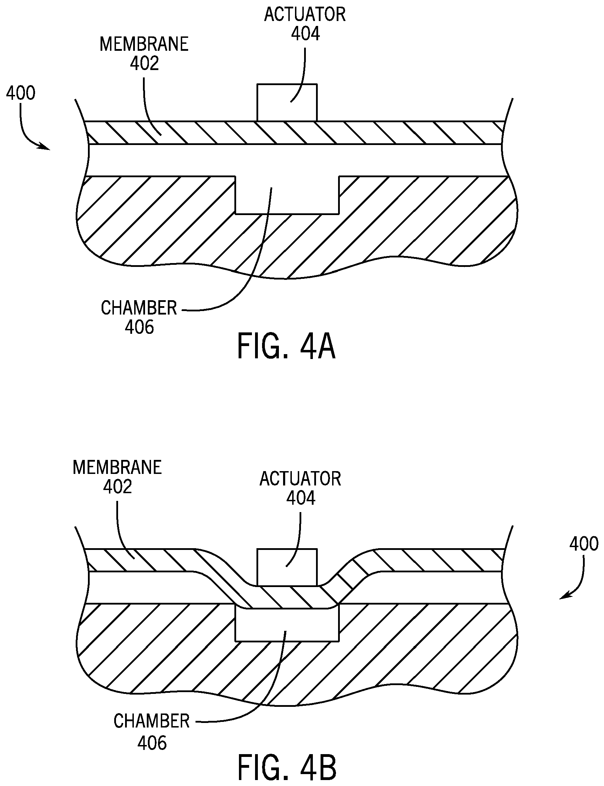

Figs. 4A and 4B depict an example MEMS device 400 that functions as an active valve in pumping unit 106. In Fig. 4A, MEMS device 400 is shown in a non-energized configuration. In Fig. 4B, actuator 404 is energized (under voltage) causing membrane 402 to contact and seat the edges of chamber 406, thereby creating a seal that prevents insulin flow.

Figs. 5A and 5B depict block diagrams of example configurations of pumping unit 104 in Fig. 1 to vary flow rate and pressure, respectively. Both configurations for pumping unit 106 comprise pumping sections and valves. In Fig. 5A, pumping unit 106 incorporates several (three) MEMS devices in parallel that function together as the pump (pumping section or element) to increase flow rate within the channel of the MEMS device. Two MEMS devices appear between the pump (pumping element) and reservoir and pump and insulin needle. These MEMS devices function as valves. In this configuration, parallel pumping chambers, separated by individual controllable valves (not shown in figure 5A) may be used to increase flow rate as a needed. For example, current flow rate may be insufficient to address dosing needs for a patient. Pumping chambers in series may be used to increase output pressures as needed. For example, pressure adjustments may be required to overcome pressure issues at the tip of the insulin needle (in a deployed configuration).

Fig. 6 depicts a cross sectional view of an example device 600 (or pod) for delivering insulin to a diabetes patient. In this implementation, reservoir 602 extends within the housing of device 600 above the active components. Reservoir 602 is defined by the outer wall 604 of the housing and an inner wall 606 that separates the reservoir 602 from the other components. Reservoir 602 communicates with channel 608 that narrows down the side of device 600 into sump 610 as shown. Sump 610 communicates with the pump/valve elements of MEMS device 612 via a narrow and short channel 614. The pump element and sensors of MEMS device 612 communicate with insulin needle 616 via an interposer. The sensors are also connected to CGM 618 to enable CGM 618 to track glucose levels in the patient as described above. MCU 620 communicates with the actuators and CGM of MEMS device 612 as described above. Battery 622 provides power to MCU 620 which in turn provides power to the actuator on the MEMS device. In this configuration, MEMS device 612 is positioned to the left of MCU 620, CGM 718 and battery 622.

Fig. 7 depicts a cross sectional view of another example device 700 (or pod) for delivering insulin to a diabetes patient. Many of the components in example device 600 are positioned similar to those in device 700. However, in this implementation, reservoir is positioned below the MEMS device, MCU and battery. (However, those skilled in the art know that the reservoir may be positioned next to or above the MEMS device.) In addition, dual needles are used. Needle 702 functions similar to needle 616 except that it does not sense glucose level (CGM). Needle 704 performs that task. The housing for device 700 is configured as a rounded dome.

As noted above, devices (102, 600, 700) are described for delivering insulin to a diabetes patient. However, these devices may be used to deliver other medications such as small molecule pharmaceutical solutions, large molecule or protein drug solutions, saline solutions, blood or other fluids known to those skilled in the art.

It is to be understood that the disclosure teaches examples of the illustrative embodiments and that many variations of the invention can easily be devised by those skilled in the art after reading this disclosure and that the scope of the present invention is to be determined by the claims below.

## Claims

1. A device (102) for delivering a medication to a patient, the device (102) configured as a wearable apparatus for managing medication delivery, the device comprising:
a reservoir (104, 200-9, 602) for storing the medication to be delivered to the patient;
a continuous glucose monitoring sensor (112, 200-8, 618) configured to be inserted into tissue of the patient for measuring glucose levels in the patient to set flow rates for medication delivery;
a needle (110, 616, 702) for delivering the medication from the reservoir into the patient; and
a pumping unit (106) including MEMS devices (106-1 and 106-2, 106-3 and 106-4, 106-5 and 106-6, 200-3, 302, 350, 380) configured to function as (a) a pump (106-3 and 106-4, 200-3, 302, 384) for pumping the medication from the reservoir (104, 200-9, 602) through a flow path for medication to the needle (110, 616, 702) and/or (b) a valve (106-1 and 106-2, 106-5 and 106-6, 200-2, 200-4, 300, 304) for regulating flow of the medication in the flow path from the reservoir (104, 200-9, 602) through the needle (110, 616, 702),
**characterised in that** the MEMS devices include a first MEMS device and second MEMS device configured in series with a common membrane (302-2) and flow path to enable medication to flow therebetween, the first MEMS device configured as a pumping section (302) for pumping the medication through the flow path and the second MEMS device configured as an active valve (300, 304) for regulating flow of the medication through the flow path from the reservoir through the needle.

2. The device for delivering of claim 1 wherein the first and second MEMS devices are configured adjacent to one another with the common membrane (302-2) and flow path to enable medication to directly flow therebetween.

3. The device for delivering of claim 1 wherein the pumping section (302) and the active valve (300, 304) each including an actuator ( 302-1, 304-1, 352) for moving or controlling flow of medication along the flow path.

4. The device for delivering of claim 1 further comprises a microcontroller unit (108) in communication with the continuous glucose monitoring sensor (112, 200-8, 618) and pumping unit (106), the microcontroller unit (108) configured to control the operation of pumping unit (106) to deliver medication from the reservoir (104, 200-9, 602) through the needle (110, 616, 702) at set flow rates over specified time intervals based on data from the continuous glucose monitoring sensor (112, 200-8, 618).

5. The device for delivering of claim 4 further comprises a battery and power controller (114) in communication with the pumping unit (106) and microcontroller unit (108) for powering the pumping unit (106) and microcontroller unit (108).

6. The device for delivering of claim 4 wherein the continuous glucose monitoring sensor (112, 200-8, 618) is powered by the battery and power controller (114) via the microcontroller unit (108).

7. The device for delivering of claim 1 further configured to communicate wirelessly with a mobile device (116) or computer for uploading flow rates, glucose levels and/or other data to the mobile device or computer.

8. The device of claim 1 wherein the medication is insulin.

9. The device of claim 1 wherein the pumping unit (106) and needle (110, 616, 702) are connected directly without tubing.

10. The device of claim 1 wherein the first and second MEMS devices having first and second chambers, respectively configured in series to control the flow rate or output pressure of the medication through the first and second chambers along the flow path delivered to the patient.

11. The device of claim 10 wherein the MEMS devices (106-1 and 106-2, 106-3 and 106-4, 106-5 and 106-6, 200-3) include a third MEMS device (300, 304) configured in series with the first and second MEMS devices with the second MEMS device located between the first MEMS device and third MEMS device, the third MEMS device configured to function as an active valve for regulating flow of the medication from the reservoir through the needle.

12. The device of claim 11 wherein the MEMS devices (106-1 and 106-2, 106-3 and 106-4, 106-5 and 106-6, 200-3) include a fourth MEMS device configured in series with the first, second and third MEMS devices to increase output pressure of the medication along the flow path delivered to the patient.

13. The device of claim 11 wherein the MEMS devices (106-1 and 106-2, 106-3 and 106-4, 106-5 and 106-6, 200-3) include a fourth MEMS devices configured in parallel with the first and second MEMS devices to increase a flow rate of the medication along the flow path delivered to the patient.

## Patentansprüche

1. Eine Vorrichtung (102) zum Abgeben eines Medikaments an einen Patienten, wobei die Vorrichtung (102) als Wearable-Apparat zur Steuerung der Medikamentenabgabe ausgebildet ist, wobei die Vorrichtung Folgendes umfasst:
einen Behälter (104, 200-9, 602) zur Lagerung des an den Patienten abzugebenden Medikaments;
einen Sensor (112, 200-8, 618) zur kontinuierlichen Glukoseüberwachung, ausgebildet, um in Gewebe des Patienten eingeführt zu werden, um Glukosespiegel beim Patienten zu messen, um Strömungsraten zur Medikamentenabgabe festzulegen;
eine Nadel (110, 616, 702) zur Abgabe des Medikaments aus dem Behälter an den Patienten; und
eine Pumpeneinheit (106), die MEMS-Vorrichtungen (106-1 und 106-2, 106-3 und 106-4, 106-5 und 106-6, 200-3, 302, 350, 380) einschließt, ausgebildet, um als Folgendes zu fungieren: (a) als Pumpe (106-3 und 106-4, 200-3, 302, 384), um das Medikament aus dem Behälter (104, 200-9, 602) durch einen Strömungspfad für das Medikament an die Nadel (110, 616, 702) zu pumpen, und/oder (b) als Ventil (106-1 und 106-2, 106-5 und 106-6, 200-2, 200-4, 300, 304) zum Regeln des Stroms des Medikaments im Strömungspfad vom Behälter (104, 200-9, 602) durch die Nadel (110, 616, 702);
**dadurch gekennzeichnet, dass** die MEMS-Vorrichtungen eine erste MEMS-Vorrichtung und eine zweite MEMS-Vorrichtung einschließen, seriell angeordnet mit einer gemeinsamen Membran (302-2) und Strömungspfad, um den Strom des Medikaments dazwischen zu ermöglichen, wobei die erste MEMS-Vorrichtung als Pumpenabschnitt (302) zum Pumpen des Medikaments durch den Strömungspfad ausgebildet ist und die zweite MEMS-Vorrichtung als Aktivklappe (300, 304) zum Regeln des Stroms des Medikaments durch den Strömungspfad vom Behälter durch die Nadel ausgebildet ist.

2. Die Vorrichtung zum Abgeben gemäß Anspruch 1, wobei die erste und die zweite MEMS-Vorrichtung nebeneinander mit der gemeinsamen Membran (302-2) und dem Strömungspfad angeordnet sind, um die direkte Strömung des Medikaments dazwischen zu ermöglichen.

3. Die Vorrichtung zum Abgeben gemäß Anspruch 1, wobei der Pumpenabschnitt (302) und die Aktivklappe (300, 304) jeweils ein Antriebselement (302-1, 304-1, 352) zum Bewegen oder Steuern des Stroms des Medikaments entlang dem Strömungspfad einschließen.

4. Die Vorrichtung zum Abgeben gemäß Anspruch 1 umfasst weiter eine Mikrocontrollereinheit (108) in Kommunikation mit dem Sensor (112, 200-8, 618) zur kontinuierlichen Glukoseüberwachung und der Pumpeneinheit (106); wobei die Mikrocontrollereinheit (108) konfiguriert ist, um den Betrieb der Pumpeneinheit (106) zu steuern, um Medikament aus dem Behälter (104, 200-9, 602) durch die Nadel (110, 616, 702) über angegebene Zeitintervalle bei festgelegten Strömungsraten abzugeben, basierend auf Daten vom Sensor (112, 200-8, 618) zur kontinuierlichen Glukoseüberwachung.

5. Die Vorrichtung zum Abgeben gemäß Anspruch 4 umfasst weiter einen Batterie- und Leistungsregler (114) in Kommunikation mit der Pumpeneinheit (106) und der Mikrocontroller-Einheit (108) zur Versorgung der Pumpeneinheit (106) und der Mikrocontroller-Einheit (108) mit Strom.

6. Die Vorrichtung zum Abgeben gemäß Anspruch 4, wobei der Sensor (112, 200-8, 618) zur kontinuierlichen Glukoseüberwachung vom Batterie- und Leistungsregler (114) über die Mikrocontroller-Einheit (108) mit Strom versorgt wird.

7. Die Vorrichtung zum Abgeben gemäß Anspruch 1, die weiter ausgebildet ist, um drahtlos mit einem mobilen Gerät (116) oder Computer zu kommunizieren, um Strömungsraten, Glukosespiegel und/oder andere Daten auf das mobile Gerät oder den Computer hochzuladen.

8. Die Vorrichtung gemäß Anspruch 1, wobei das Medikament Insulin ist.

9. Die Vorrichtung gemäß Anspruch 1, wobei die Pumpeneinheit (106) und die Nadel (110, 616, 702) direkt, ohne Schlauchverbindung, verbunden sind.

10. Die Vorrichtung gemäß Anspruch 1, wobei die erste und die zweite MEMS-Vorrichtung erste und zweite Kammern haben, die jeweils seriell konfiguriert sind, um die Strömungsrate oder den Ausgangsdruck des Medikaments durch die ersten und zweiten Kammern entlang dem Strömungspfad zu steuern, der an den Patienten abgegeben wird.

11. Die Vorrichtung gemäß Anspruch 10, wobei die MEMS-Vorrichtungen (106-1 und 106-2, 106-3 und 106-4, 106-5 und 106-6, 200-3) eine dritte MEMS-Vorrichtung (300, 304) einschließen, seriell mit der ersten und der zweiten MEMS-Vorrichtung angeordnet, wobei sich die zweite MEMS-Vorrichtung zwischen der ersten und der dritten MEMS-Vorrichtung befindet, wobei die dritte MEMS-Vorrichtung ausgebildet ist, um als Aktivklappe zum Regeln des Stroms des Medikaments vom Behälter durch die Nadel zu fungieren.

12. Die Vorrichtung gemäß Anspruch 11, wobei die MEMS-Vorrichtungen (106-1 und 106-2, 106-3 und 106-4, 106-5 und 106-6, 200-3) eine vierte MEMS-Vorrichtung einschließen, seriell mit der ersten, zweiten und dritten MEMS-Vorrichtung angeordnet, um den Ausgangsdruck des Medikaments entlang dem dem Patienten verabreichten Strömungspfad zu erhöhen.

13. Die Vorrichtung gemäß Anspruch 11, wobei die MEMS-Vorrichtungen (106-1 und 106-2, 106-3 und 106-4, 106-5 und 106-6, 200-3) eine vierte MEMS-Vorrichtung einschließen, parallel zu der ersten und der zweiten MEMS-Vorrichtung angeordnet, um eine Strömungsrate des Medikaments entlang dem dem Patienten verabreichten Strömungspfad zu erhöhen.

## Revendications

1. Dispositif (102) destiné à administrer un médicament à un patient, le dispositif (102) étant configuré comme un appareil portable pour gérer administration du médicament, ledit dispositif comprenant :
un réservoir (104, 200-9, 602) destiné à stocker le médicament à administrer au patient ;
un capteur de surveillance continue de la glycémie (112, 200-8, 618) conçu pour être inséré dans la tissus du patient afin de mesurer taux de glucose dans le patient pour régler les débits d'administration du médicament ;
une aiguille (110, 616, 702) destinée à administrer le médicament depuis le réservoir dans le patient ; et
une unité de pompage (106) comprenant des dispositifs MEMS (106-1 et 106-2, 106-3 et 106-4, 106-5 et 106-6, 200-3, 302, 350, 380) configurés pour fonctionner comme (a) une pompe (106-3 et 106-4, 200-3, 302, 384) pour pomper le médicament depuis le réservoir (104, 200-9, 602) à travers un trajet d'écoulement du médicament vers l'aiguille (110, 616, 702) et/ou (b) une vanne (106-1 et 106-2, 106-5 et 106-6, 200-2, 200-4, 300, 304) destiné à réguler le débit du médicament dans le trajet d'écoulement depuis le réservoir (104, 200-9, 602) à travers l'aiguille (110, 616, 702),
**caractérisé en ce que** les dispositifs MEMS comprennent un premier dispositif MEMS et un deuxième dispositif MEMS configurés en série avec une membrane commune (302-2) et trajet d'écoulement commun afin de permettre au médicament de s'écouler entre eux, le premier dispositif MEMS étant configuré comme une section de pompage (302) destinée à pomper le médicament à travers le trajet d'écoulement et le deuxième dispositif MEMS étant configuré comme une valve active (300, 304) destinée à réguler l'écoulement du médicament à travers le trajet d'écoulement depuis le réservoir à travers l'aiguille.

2. Dispositif d'administration selon la revendication 1, où les premier et deuxième dispositifs MEMS sont disposés de manière adjacente l'un à l'autre, avec la membrane commune (302-2) et le trajet d'écoulement, de manière à permettre au médicament de s'écouler directement entre eux.

3. Dispositif d'administration selon la revendication 1, où la section de pompage (302) et la valve active (300, 304) comprennent chacune un actionneur (302-1, 304-1, 352) destiné à déplacer ou à contrôler l'écoulement du médicament le long du trajet d'écoulement.

4. Le dispositif d'administration selon la revendication 1 comprend en outre une unité de microcontrôleur (108) en communication avec le capteur de surveillance continue de la glycémie (112, 200-8, 618) et l'unité de pompage (106), l'unité de microcontrôleur (108) étant configurée pour commander le fonctionnement de l'unité de pompage (106) afin d'administrer le médicament depuis le réservoir (104, 200-9, 602) par l'intermédiaire de l'aiguille (110, 616, 702) à des débits définis sur des intervalles de temps spécifiés, en fonction des données provenant du capteur de surveillance continue de la glycémie (112, 200-8, 618).

5. Le dispositif d'administration selon la revendication 4 comprend en outre une batterie et un contrôleur d'alimentation (114) en communication avec l'unité de pompage (106) et l'unité de microcontrôleur (108) pour alimenter l'unité de pompage (106) et l'unité de microcontrôleur (108).

6. Dispositif d'administration selon la revendication 4, où le capteur de surveillance continue de la glycémie (112, 200-8, 618) est alimenté par la batterie et le contrôleur d'alimentation (114) via l'unité de microcontrôleur (108).

7. Le dispositif d'administration selon la revendication 1, configuré en outre pour communiquer sans fil avec un appareil mobile (116) ou un ordinateur afin de transférer les débits, les taux de glucose et/ou d'autres données vers l'appareil mobile ou l'ordinateur.

8. Le dispositif selon la revendication 1, où le médicament est l'insuline.

9. Dispositif selon la revendication 1, où l'unité de pompage (106) et l'aiguille (110, 616, 702) sont reliées directement, sans tubulure.

10. Dispositif selon la revendication 1, où les premier et deuxième dispositifs MEMS comportent des première et deuxième chambres, respectivement configurées en série pour contrôler le débit ou la pression de sortie du médicament à travers les première et deuxième chambres le long du trajet d'écoulement vers le patient.

11. Dispositif selon la revendication 10, où les dispositifs MEMS (106-1 et 106-2, 106-3 et 106-4, 106-5 et 106-6, 200-3) comprennent un troisième dispositif MEMS (300, 304) monté en série avec les premier et deuxième dispositifs MEMS, le deuxième dispositif MEMS étant situé entre le premier dispositif MEMS et le troisième dispositif MEMS, le troisième dispositif MEMS étant configuré pour fonctionner comme une valve active destinée à réguler l'écoulement du médicament depuis le réservoir à travers l'aiguille.

12. Le dispositif selon la revendication 11, où les dispositifs MEMS (106-1 et 106-2, 106-3 et 106-4, 106-5 et 106-6, 200-3) comprennent un quatrième dispositif MEMS monté en série avec les premier, deuxième et troisième dispositifs MEMS afin d'augmenter la pression de sortie du médicament le long du trajet d'écoulement vers le patient.

13. Dispositif selon la revendication 11, où les dispositifs MEMS (106-1 et 106-2, 106-3 et 106-4, 106-5 et 106-6, 200-3) comprennent un quatrième dispositif MEMS configuré en parallèle avec les premier et deuxième dispositifs MEMS afin d'augmenter le débit du médicament le long du trajet d'écoulement vers le patient.
